# EUROPÄISCHE PATENTANMELDUNG

(11) **EP 3 770 012 A2**
(43) Veröffentlichungstag der Anmeldung: **27.01.2021**
(21) Anmeldenummer: 20185096.3
(22) Anmeldetag: 09.07.2020
(51) Int. Cl.: B60N 2/75, B60R 7/04

(54) **ARMLEHNE FÜR EINEN SITZ**

(30) Priorität: 10.07.2019 DE 102019004759
(71) Anmelder: GRAMMER AG, 92289 Ursensollen (DE)
(72) Erfinder: Nuss, Ralph, 92284 Poppenricht (DE); Keller, Hubert, 92729 Weiherhammer (DE)
(74) Vertreter: Roche, von Westernhagen & Ehresmann

(57) **Zusammenfassung**

Die Erfindung betrifft Armlehne (10) für einen Sitz, insbesondere für einen Fahrzeugsitz, mit einer Basis (11), welcher wenigstens ein Behälter (18) mit einem Innenraum (27) zugeordnet ist, mit einer relativ zu dem Behälter (18) zwischen einer unteren Endposition und einer oberen Endposition schwenkbaren Armauflage (12) mit einer Armauflagefläche (13), wobei der Armauflage (12) wenigstens eine Gehäusewand (19a, 19b, 19c) zugeordnet ist, die mit der Armauflage (12) derart bewegungsverbunden ist, dass sie einen beim Aufschwenken der Armauflage (12) zwischen einer Anschlagfläche (26) der Armauflage (12) und einer Auflagefläche (25) an dem Basisteil (11) entstehenden Zwischenraum verschließt, wobei die Armauflage (12) eine Öffnung (16) aufweist, durch welche der Zugriff auf den Innenraum (27) möglich ist.

## Beschreibung

Die Erfindung betrifft eine Armlehne für einen Sitz, insbesondere den Sitz eines Fahrzeugs. Fahrzeug kann im Sinne der Erfindung ein Luft-, Land- oder Wasserfahrzeug sein.

Eine Armlehne ist aus der DE 101 10 330 A1 bekannt. Die Armlehne ist Teil einer Mittelkonsole und umfasst ein Armauflageteil, welches nach vorne und nach hinten verstellbar ist und eine Öffnung aufweist, die mittels eines Deckels verschlossen ist, der zwischen einer offenen und einer geschlossenen Position bewegbar ist. Die Armauflage ist an einem höhenverstellbar gelagerten Rahmen gehalten, in welchem ein Ablagefach ausgebildet ist.

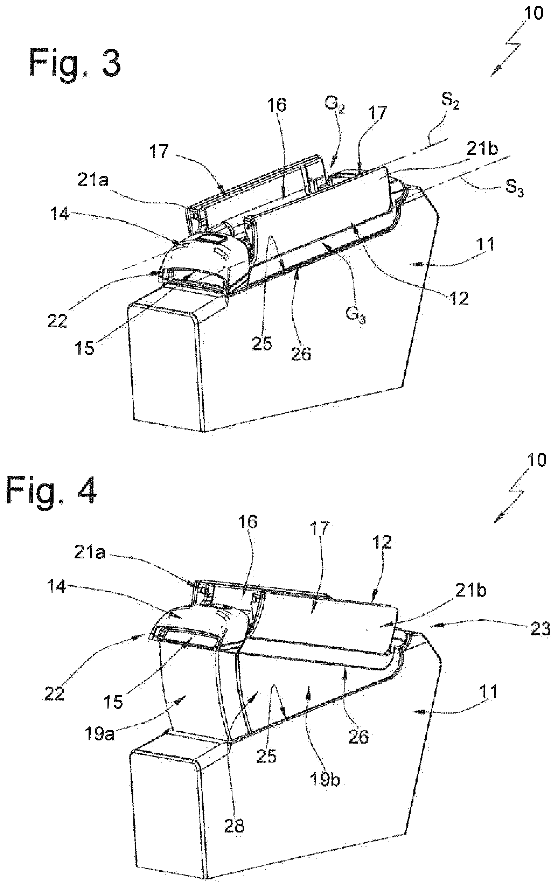

In der DE 100 32 657 A1 ist eine Mittelkonsole mit einer Armlehne beschrieben. Unter der Armlehne ist ein erstes Staufach sowie ein zweites Staufach ausgebildet. Eine Armauflage ist um eine Schwenkachse schwenkbar und kann derart geöffnet werden, dass auf das erste oder auf das zweite Staufach zugegriffen werden kann. Bei einem in Fig. 4 dargestellten Ausführungsbeispiel ist die Armauflage einem äußeren Gehäuse 30 zugeordnet, welches gegenüber einem inneren Gehäuse 28 vertikal verstellbar ist. Die Verstellung erfolgt mittels Führungen und kann eine geradlinige oder gekrümmte Bahn bilden.

Aus der DE 196 43 051 C1 ist ein abdeckbarer Behälter für die sitzseitige Zuordnung in Fahrzeugen offenbart. Der Behälter umfasst ein Gehäuse 10 mit einer Gehäuseöffnung 101, die von einer Armauflage 11 verschließbar ist. Die Armauflage 11 ist schwenkbar gelagert und kann in unterschiedlichen Schwenkpositionen arretiert werden.

Aus der DE 10 2008 005 642 A1 ist eine Armlehne bekannt, die in einer Mittelkonsole eines Kraftfahrzeuges ausgebildet ist. Die Armlehne umfasst ein Aufnahmefach, welches in einem schwenkbaren Gehäuse angeordnet ist. Das Aufnahmefach kann mittels eines schwenkbaren Deckels verschlossen werden. Dem Gehäuse ist außerdem ein Bedienfeld zugeordnet, welches ebenfalls mit dem Gehäuse verschwenkbar ist.

Es war Aufgabe der Erfindung, eine Armlehne zu schaffen, die eine einfache Konstruktion aufweist und wobei eine Armauflage in unterschiedliche Positionen einstellbar ist.

Die Aufgabe wurde gelöst durch eine Armlehne mit den Merkmalen des Anspruchs 1.

Die Armlehne umfasst ein Basisteil, welches relativ zu dem Fahrzeug fest angeordnet ist. Das Basisteil kann z.B. von einer Mittelkonsole gebildet sein. Alternativ kann das Basisteil z.B. von einem Rahmen oder einem Gehäuse gebildet sein, das fahrzeugfest angeordnet ist. Dem Basisteil ist ein Behälter zugeordnet, der einen Innenraum bildet, in welchem Gegenstände aufgenommen werden können. Der Behälter weist eine Öffnung auf, über die der Innenraum zugänglich ist.

An dem Basisteil ist eine Armauflage relativ zu dem Behälter schwenkbar gehalten. Die Armauflage bildet z.B. mit dem Basisteil ein Schwenkgelenk. Die Armauflage ist zwischen einer unteren Endposition und einer oberen Endposition schwenkbar. In der unteren Endposition liegt eine Anschlagfläche der Armauflage an einer Auflagefläche des Basisteils an. Wenn die Armauflage aus der unteren Endposition in Richtung obere Endposition bewegt wurde, ist die Anschlagfläche von der Auflagefläche beabstandet. In der oberen Endposition wirken z.B. eine Anschlagfläche der Armauflage und eine Anschlagfläche des Basisteils oder eines anderen fahrzeugfesten Teils zusammen.

Die Armlehne umfasst eine Verriegelungsvorrichtung mit welcher die Armauflage in stufenweise oder stufenlos in unterschiedlichen Schwenkpositionen lösbar verriegelbar ist. Mittels einer Betätigung kann die Verriegelungsvorrichtung zwischen einer Riegelposition und einer Löseposition verstellt werden. Die Verriegelungsvorrichtung umfasst erste, der Basis zugeordnete Verriegelungsmittel und zweite der Armauflage zugeordnete Verriegelungsmittel, die lösbar in Eingriff gebracht werden können.

Der Armauflage sind Gehäusewände zugeordnet, die einen beim Aufschwenken der Armauflage zwischen der Armauflage und dem Behälter entstehenden Zwischenraum verschließen. D.h., dass ein bei der Bewegung der Armauflage aus einer unteren Endposition, in eine Position, in welcher die Armauflage um einen bestimmten Schwenkwinkel in Richtung der oberen Endposition geschwenkt ist, der Zwischenraum zwischen der Armauflage und dem Behälter von den Gehäusewänden verschlossen wird, so dass ein zusätzlicher Raum geschaffen wird, welcher einen Tunnel bildet, der eine Öffnung der Armauflage mit dem Innenraum des Behälters verbindet.

Der Zwischenraum kann z.B. zwischen einer Anschlagfläche der Armauflage und einer Auflagefläche der Basis gebildet sein. In der unteren Endposition stehen dann die Anschlagfläche und die Auflagefläche in Kontakt. Auf diese Weise kann der Innenraum des Behälters lediglich über die Öffnung erreicht werden.

Gemäß einer Ausführungsform kann die Öffnung der Armauflage mittels eines zwischen einer Offenposition und einer Schließposition bewegbaren Deckels verschlossen werden. Der Deckel ist z.B. schwenkbar an der Armauflage gelagert. Der Deckel kann einen oder mehrere Teile aufweisen. Der Deckel kann in jeder Position der Armauflage zwischen der Offenposition und der Schließposition bewegbar sein.

Die Öffnung ist z.B. in einem Bereich der Armauflagefläche der Armauflage angeordnet.

Eine Außenfläche des Deckels bildet z.B. wenigstens einen Teil der Armauflagefläche. Z.B. kann die gesamte Armauflagefläche von der Außenfläche des Deckels gebildet sein. Alternativ ist z.B. lediglich ein Teil der Außenfläche der Armauflage von der Außenfläche des Deckels gebildet.

Z.B. ist in der Basis wenigstens ein Schacht ausgebildet, in welchem die Gehäusewände wenigstens teilweise verstaubar sind. Auf diese Weise treten die Gehäusewände in der untersten Position der Armauflage nicht in Erscheinung, sondern bewegen sich erst beim Aufschwenken der Armauflage aus dem Schacht hinaus.

Die Gehäusewand grenzt z.B. den Innenraum bzgl. wenigstens drei Raumrichtungen ab. Die Gehäusewand kann von separaten Teilen oder von einem Teil gebildet sein.

Die Gehäusewand bildet z.B. einen Tunnel derart, dass der Innenraum des Behälters lediglich über die Öffnung der Armauflage zugänglich ist. Der Tunnel verbindet z.B. die Öffnung der Armauflage und die Öffnung des Behälters miteinander.

Der Deckel kann z.B. einteilig ausgebildet sein oder weist z.B. wenigstens zwei Deckelteile auf, die gemeinsam oder separat bewegbar sind.

Die Armauflage ist z.B. zwischen der unteren Endposition und der oberen Endposition über einen Schwenkwinkel von etwa 20° bis 45° schwenkbar.

An einem vorderen, der Schwenkachse abgewandten Endbereich weist die Armauflage z.B. ein Bedienfeld zum Schalten und Steuern von fahrzeuginternen und / oder fahrzeugexternen Geräten auf.

Ein Ausführungsbeispiel der Erfindung ist in der nachfolgenden Figurenbeschreibung, auch unter Bezugnahme auf die schematischen Zeichnungen, beispielhaft beschrieben. Dabei werden der Übersichtlichkeit halber - auch soweit unterschiedliche Ausführungsbespiele betroffen sind - gleiche oder vergleichbare Teile oder Elemente oder Bereiche mit gleichen Bezugszeichen, teilweise unter Hinzufügung kleiner Buchstaben, bezeichnet.

Alle offenbarten Merkmale sind für sich erfindungswesentlich. In die Offenbarung der Anmeldung wird hiermit auch der Offenbarungsinhalt der zitierten Druckschriften und der beschriebenen Vorrichtungen des Standes der Technik inhaltlich vollumfänglich mit einbezogen, auch zu dem Zweck, einzelne oder mehrere Merkmale der dort offenbarten Gegenstände in einen oder in mehrere Ansprüche der vorliegenden Anmeldung mit aufzunehmen. Auch solche geänderten Ausführungsbeispiele sind - auch wenn sie in den Zeichnungen nicht dargestellt sind - von der Erfindung mit umfasst.

Es zeigen:
Fig. 1 eine perspektivische Darstellung der Armlehne, wobei sich die Armauflage in einer unteren Position befindet und der Deckel in einer Schließposition angeordnet ist,
Fig. 2 die Armlehne gemäß Fig. 1, wobei sich die Armauflage in einer oberen Position befindet,
Fig. 3 die Armlehne gemäß Fig. 1, wobei sich ein Deckel in einer Offenstellung befindet,
Fig. 4 die Armlehne gemäß Fig. 2, wobei sich der Deckel in der Offenstellung befindet.
Fig. 5 eine Seitenansicht der Armlehne gemäß Fig. 1,
Fig. 6 eine Seitenansicht der Armlehne gemäß Fig. 2,
Fig. 7 eine Ansicht gemäß Ansichtspfeil A in Fig. 5,
Fig. 8 eine Schnittdarstellung gemäß Schnittlinie B-B in Fig. 7,
Fig. 9 eine Ansicht gemäß Ansichtspfeil C in Fig. 6,
Fig. 10 eine Schnittdarstellung gemäß Schnittlinie D-D in Fig. 9.

Eine Armlehne insgesamt ist in den Figuren mit dem Bezugszeichen 10 bezeichnet.

Die Armlehne 10 ist z.B. derart in einem Fahrzeug angeordnet, dass sich eine Längsmittelachse m der Armlehne 10 parallel zu den Richtungen x1 und x2 erstreckt, wobei die Richtungen x1 und x2 den Vorwärts- bzw. Rückwärtsfahrtrichtungen entsprechen. Die Armlehne 10 kann z.B. zwischen zwei Sitzen einer vorderen Sitzreihe oder zwischen zwei Sitzen einer hinteren Sitzreihe des Fahrzeugs angeordnet sein. Es kommen aber alternativ auch andere Anordnungen und Orientierungen der Armlehne 10 in Betracht.

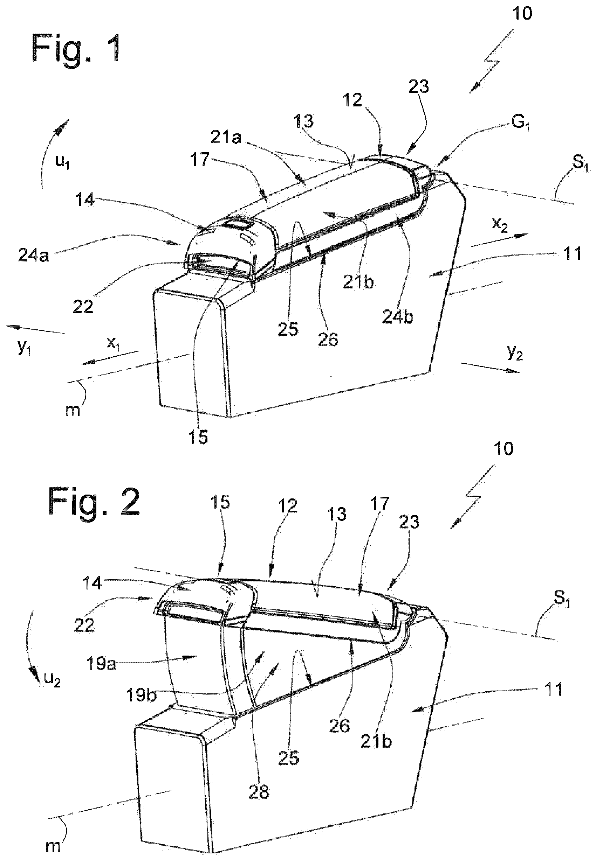

Die Armlehne 10 umfasst eine Basis 11, die im vorliegenden Beispiel Teil einer Mittelkonsole ist. Eine Armauflage 12 ist um eine Schwenkachse S schwenkbar an der Basis 11 gehalten und bildet mit der Basis 11 ein Schwenkgelenk G1. In Fig. 1 ist die Armauflage 12 in der unteren Endposition dargestellt. Fig. 2 zeigt die Armlehne 10 mit einer in der oberen Endposition befindlichen Armauflage 12. Aus der unteren Endposition gemäß Fig. 1 ist die Armauflage 12 in Richtung u1 in die obere Endposition schwenkbar. Aus der oberen Endposition gemäß Fig. 2 ist die Armauflage 12 in Richtung u2 in die untere Endposition schwenkbar.

Ein hinterer Endbereich 23 der Armauflage befindet sich nahe dem Schwenkgelenk G1, während ein vorderer Endbereich 22 an einer bezüglich einer Mittelachse m von dem Gelenk G1 abgewandten Seite der Armauflage 12 befindet.

Die Armauflage 12 umfasst eine Armauflagefläche 13, auf welcher ein Fahrzeuginsasse seinen Arm abstützen kann. Ein Bedienfeld 14 ist in einem vorderen Bereich der Armauflagefläche 13 angeordnet. An dem Bedienfeld 14 können diverse Bedienelemente angesteuert werden, mit denen unterschiedliche Fahrzeugfunktionen oder gesonderte Geräte wie Telefon, fahrzeuginterne oder -externe Musikgeräte etc. geschaltet oder gesteuert werden können.

Außerdem umfasst das Bedienfeld 14 eine Betätigung einer Verriegelungsvorrichtung 15 der Armlehne 10. Die Betätigung erfolgt hier an einem vorderen Ende des Bedienfeldes. In der oberen und der unteren Endposition sowie in Zwischenpositionen kann die Armauflage 12 mittels der Verriegelungsvorrichtung 15 lösbar verriegelt werden. Die Verriegelungsvorrichtung 15 umfasst hier nicht gezeigte erste, der Basis 11 zugeordnete Verriegelungselemente und zweite, der Armauflage 12 zugeordnete Verriegelungselemente. Die Gestaltung der Verriegelung sind dem Fachmann bekannt. Auf die Verriegelung soll hier nicht weiter eingegangen werden.

Die Armauflage 12 umfasst gemäß Fig. 3 eine Öffnung 16, die mittels eines Deckels 17 verschließbar ist. In jeder Schwenkposition der Armauflage 12 kann der Deckel 17 zwischen einer Schließposition (siehe z.B. Fig. 1) und einer Offenposition (siehe z.B. Fig. 3) bewegt werden. Auf die Art des Deckels 17 kommt es bei der Erfindung nicht an. Es können auch andere, als der hier beschriebene Deckel 17 verwendet werden.

Bei dem vorliegenden Ausführungsbeispiel ist der Deckel 17 ein sogenannter Schmetterlingsdeckel, welcher zwei Deckelteile 21a und 21 b umfasst. Der Deckelteil 21a ist um eine Schwenkachse S2 schwenkbar (siehe Fig. 3), die sich parallel zu der Mittelachse m erstreckt und in einem Seitenbereich 24a der Armauflage 12 angeordnet ist. Der Deckelteil 21a bildet mit der Basis 11 ein Schwenkgelenk G2. Der Deckelteil 21b ist um eine Schwenkachse S3 schwenkbar, die sich ebenfalls parallel zu der Mittelachse m erstreckt und in einem Seitenbereich 24b der Armauflage 12 angeordnet ist. Der Deckelteil 21b bildet mit der Basis 11 ein Schwenkgelenk G3.

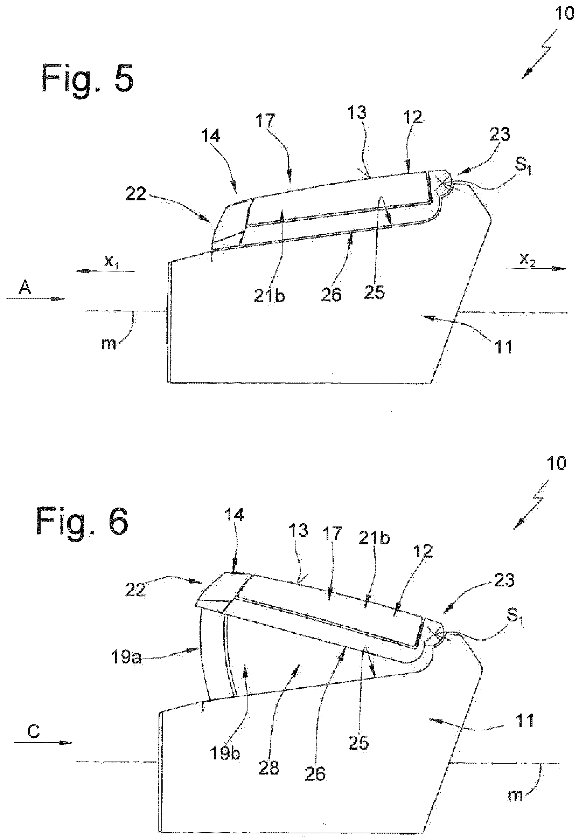

Gemäß der Fig. 5 und 6 liegt in der unteren Endposition der Armauflage 12 eine Anschlagfläche 25 der Armauflage 12 an einer Auflagefläche 26 der Basis 11 an. Wie in den Fig. 2, 4, 6 und 10 zu erkennen ist, ist die Anschlagfläche 25 in der oberen Endposition sowie in Zwischenpositionen zwischen der unteren Endposition und der oberen Endposition von der Auflagefläche 26 beabstandet. Ein Zwischenraum zwischen der Auflagefläche 26 und der Anschlagfläche 25 ist von Gehäusewänden 19a, 19b und 19c verdeckt.

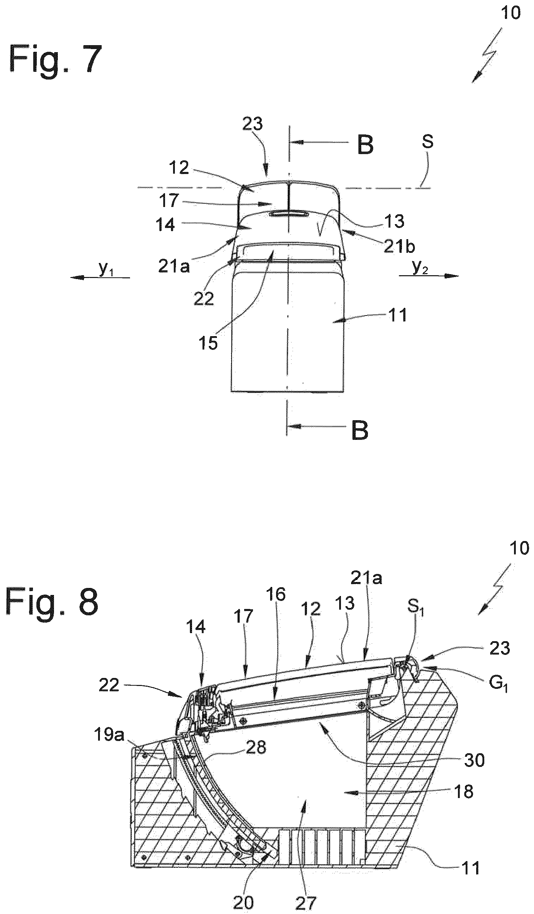

Gemäß der Fig. 8 umfasst die Basis 11 einen Behälter 18 mit einem Innenraum 27 und einer dem Deckel 17 zugewandten Öffnung 30. Der Innenraum 27 ist über die Öffnung 16 des Deckels und die Öffnung 30 des Behälters 18 zugänglich, wenn sich der Deckel 17 in seiner Offenposition befindet. In der Schließposition des Deckels 17 ist der Innenraum 27 vollständig abgeschlossen und für einen Beutzer unzugänglich.

Die Gehäusewand 19a bildet eine Abtrennung des Innenraums 27 in Richtung x1 und die Gehäusewände 19b und 19c bilden eine Abtrennung in die Richtungen y1 und y2. Auf diese Weise entsteht zwischen den Gehäusewänden 19a, 19b und 19c, dem Schwenkgelenk G1 und zwischen der Armauflage 12 und dem Behälter 18 ein zusätzlicher Raum, welcher einen Tunnel 29 zu dem Innenraum 27 bildet, welcher über die Öffnung 16 zugänglich ist. Die Gehäusewände 19b und 19c sind etwa rechtwinklig zu der Gehäusewand 19a ausgebildet.

Alle Gehäusewände 19a, 19b und 19c sind an der Armauflage 12 befestigt und mit dieser bewegungsverbunden. Die Gehäusewand 19a ist an dem vorderen Endbereich 22 und die Gehäusewände 19b und 19c sind an den Seitenbereichen 24a und 24b der Armauflage 12 befestigt. Gemäß dem Ausführungsbeispiel sind die Gehäusewände 19a, 19b und 19c von einem Teil gebildet. Sie können alternativ aber auch separat ausgebildet sein.

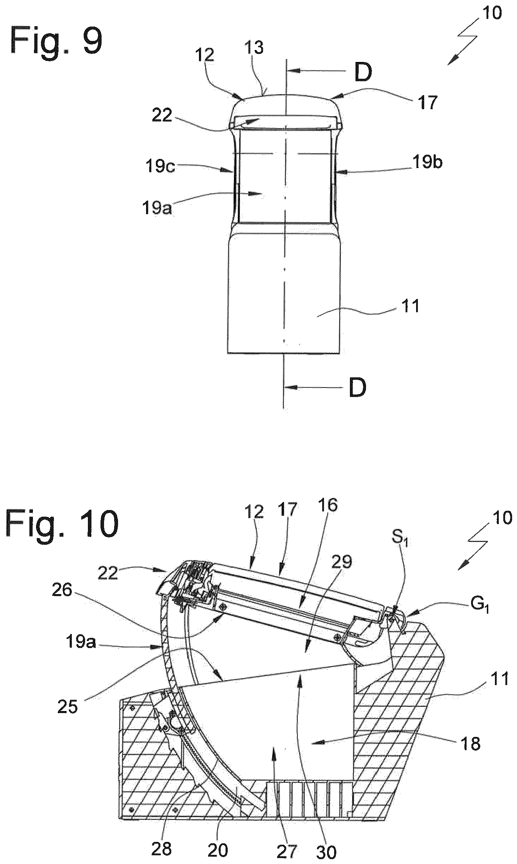

Jeder Gehäusewand 19a, 19b und 19c ist ein Schacht 20 zugeordnet, von denen lediglich ein Schacht 20 in den Fig. 8 und 10 erkennbar ist, der als Stauraum für die Gehäusewand 19a dient. In allen Positionen der Armauflage 12 zwischen der unteren Endposition und der oberen Endposition ist wenigstens ein Bereich jeder Gehäusewand 19a, 19b und 19c in dem ihr zugeordneten Schacht 20 angeordnet. Damit verschließt jede Gehäusewand 19a, 19b und 19c den Innenraum 27 des Behälters 18 und bildet den Tunnel 29 aus.

Jeder Schacht 20 bildet eine Wand 28 zu dem Innenraum 27 aus, so dass in dem Innenraum 27 befindliche Teile nicht in Kontakt mit den Gehäusewänden 19a, 19b und 19c geraten können und dadurch beschädigt werden können oder die Bewegbarkeit der Armauflage 12 beeinträchtigt werden kann.

## Patentansprüche

1. Armlehne (10) für einen Sitz, insbesondere für einen Fahrzeugsitz, mit einer Basis (11), welcher wenigstens ein Behälter (18) mit einem Innenraum (27) zugeordnet ist mit einer relativ zu dem Behälter (18) zwischen einer unteren Endposition und einer oberen Endposition schwenkbaren Armauflage (12) mit einer Armauflagefläche (13), wobei der Armauflage (12) wenigstens eine Gehäusewand (19a, 19b, 19c) zugeordnet ist, die mit der Armauflage (12) derart bewegungsverbunden ist, dass sie einen beim Aufschwenken der Armauflage (12) zwischen einer Anschlagfläche (26) der Armauflage (12) und einer Auflagefläche (26) an dem Basisteil (11) entstehenden Zwischenraum verschließt, wobei die Armauflage (12) eine Öffnung (16) aufweist, durch welche der Zugriff auf den Innenraum (27) möglich ist.

2. Armlehne nach Anspruch 1, **dadurch gekennzeichnet, dass** die Öffnung (16) mittels eines zwischen einer Offenposition und einer Schließposition bewegbaren Deckels (17) verschlossen werden kann.

3. Armlehne nach Anspruch 1 oder 2, **dadurch gekennzeichnet, dass** die Öffnung (16) in einem Bereich der Armauflagefläche (13) angeordnet ist.

4. Armlehne nach einem der vorangehenden Ansprüche, **dadurch gekennzeichnet, dass** eine Außenfläche des Deckels (17) wenigstens einen Teil der Armauflagefläche (13) bildet.

5. Armlehne nach einem der vorangehenden Ansprüche, **dadurch gekennzeichnet, dass** in der Basis (11) wenigstens ein Schacht (20) ausgebildet ist, in welchem in jeder Position zwischen der unteren Endposition und der oberen Endposition der Armauflage (12) ein Bereich der Gehäusewände (19a, 19b, 19c) aufgenommen ist.

6. Armlehne nach einem der vorangehenden Ansprüche, **dadurch gekennzeichnet, dass** der Schacht (20) eine Wand (28) zu dem Innenraum (27) bildet.

7. Armlehne nach einem der vorangehenden Ansprüche, **dadurch gekennzeichnet, dass** die Gehäusewände (19a, 19b, 19c) einteilig oder separat ausgebildet sind.

8. Armlehne nach einem der vorangehenden Ansprüche, **dadurch gekennzeichnet, dass** der Deckel zwei Deckelteile (21a, 21b) aufweist, wobei jeder Deckelteil (21a, 21b) um eine Schwenkachse schwenkbar ist, die sich parallel zu einer Längsachse (m) der Armlehne erstreckt.

9. Armlehne nach einem der vorangehenden Ansprüche, **dadurch gekennzeichnet, dass** die Armauflage (11) zwischen der unteren Position und der oberen Position in einem Schwenkwinkel von etwa 45° bewegbar ist.

10. Armlehne nach einem der vorangehenden Ansprüche, **dadurch gekennzeichnet, dass** in einem vorderen Endbereich (22) der Armauflage (12) ein Bedienfeld angeordnet ist, an welchem der Sitzinsasse Bedienelemente ansteuern kann.

11. Armlehne nach einem der vorangehenden Ansprüche, **dadurch gekennzeichnet, dass** die Gehäusewand (19a, 19b, 19c) einen Tunnel (29) bildet derart, dass der Innenraum (27) lediglich über die Öffnung (16) der Armauflage (12) und den Tunnel (29) zugänglich ist.
